(19) Europäisches Patentamt
European Patent Office
Office européen des brevets

(11) Numéro de publication: **0 482 345 B1**

(12) **FASCICULE DE BREVET EUROPEEN**

(45) Date de publication de fascicule du brevet: **07.12.94**

(51) Int. Cl.5: **A23L 1/054**, A23L 1/0532, A23L 1/305, A61K 47/36, A23L 1/09, A23L 1/302

(21) Numéro de dépôt: **91115624.8**

(22) Date de dépôt: **14.09.91**

(54) **Composition nutritionnelle et procédé de préparation.**

(30) Priorité: **26.10.90 CH 3430/90**

(43) Date de publication de la demande:
**29.04.92 Bulletin 92/18**

(45) Mention de la délivrance du brevet:
**07.12.94 Bulletin 94/49**

(84) Etats contractants désignés:
**AT BE DE DK ES FR GB GR IT LU NL SE**

(56) Documents cités:
**EP-A- 0 037 300**
**EP-A- 0 139 570**
**US-A- 3 697 287**
**US-A- 4 497 800**

**PATENT ABSTRACTS OF JAPAN, vol. 7, no. 135 (C-170)[1280], 11 juin 1983; & JP-A-58 047 463 (RAION) 19-03-1983**

**PATENT ABSTRACTS OF JAPAN, vol. 5, no. 178 (C-78)[850], 4 novembre 1981; & JP-A-56 102 766 (AJINOMOTO) 17-08-1981**

(73) Titulaire: **CLINTEC NUTRITION COMPANY**
**Three Parkway North,**
**Suite 500**
**Deerfield, Illinois 60015 (US)**

(72) Inventeur: **Masson, Gérard**
**12, Rte de Lausanne**
**CH-1096 Cully (CH)**

(74) Mandataire: **Ledzion, Andrzej et al**
**Avenue Nestlé 55**
**CH-1800 Vevey (CH)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention a trait à une composition nutritionnelle se présentant sous forme d'une émulsion huile-dans-eau pouvant être stérilisée et utilisable en alimentation entérale, ainsi qu'à un procédé pour sa préparation.

Il est connu, par exemple par le brevet US 3697287, de préparer des compositions nutritionnelles susceptibles de fournir les éléments nutritifs essentiels exigés par le corps humain, contenant des acides aminés et/ou une source d'acides aminés telle que des protéines, des glucides et des lipides, ainsi qu'un émulsifiant et éventuellement des vitamines et des sels minéraux. Bien que ces compositions possèdent une haute valeur nutritive et qu'elles soient utilisables comme supplément ou produit de remplacement des produits alimentaires chez des êtres humains en bonne santé, elles sont principalement destinées aux traitements préopératoire et/ou postopératoire des patients, ou encore à des patients chez lesquels se posent des problèmes de digestion. Ces compositions étant généralement destinées à être administrées par voie entérale, en particulier à l'aide d'une sonde nasogastrique, il est important qu'elles se présentent sous forme homogène et stable. Or, un des principaux problèmes posés par ces compositions est leur inaptitude à former des émulsions aqueuses stables pendant une période prolongée et particulièrement après avoir été stérilisées, même avec l'aide d'émulsifiant.

Une solution à ce problème a été proposée par le brevet US 4497800, et consiste à former une émulsion comprenant, outre une source de protéines, des lipides et des glucides, un agent stabilisateur consistant en un mélange particulier de diacetyl tartrate de mono- et diglycérides et de carraghénane. L'émulsion ainsi formée est ensuite stérilisée, par exemple à 90 -140 °C pendant une dizaine de secondes, et peut conserver ses qualités physique et nutritive pendant une longue période. Un inconvénient de ce procédé est toutefois l'emploi d'agents stabilisateurs synthétiques.

La demanderesse s'est posé le problème de préparer de manière industrielle une composition à haute teneur nutritive et qui conserve ses propriétés de stabilité tant physique et chimique que nutritionnelle pendant une longue période, ce même après stérilisation, sans avoir à employer des agents stabilisateurs synthétiques.

Par stabilité physique, on entend une composition de structure et de texture apparemment homogènes, et qui ne présente ni crémage, ni floculation, ni sédimentation, ni coagulation, ni formation de sérum, ni coloration, après stockage à température ambiante, pendant une longue période.

S'agissant de l'emploi d'agents stabilisateurs d'origine naturelle dans des émulsions de type huile-dans-eau, le document JP-A-56102766 suggère l'emploi d'agents épaississants tels que gommes xanthanes, locustbean, dextrines hydrogénées, carbométhoxy-cellulose ou carraghénane. Des émulsions concernées sont en fait des compositions aromatisantes à base d'extraits de viande, contenant de fortes proportions de sel. De telles émulsions ne sont pas sujettes à stérilisation.

JP-A-58047463 décrit également l'emploi de carraghénanes et/ou de gommes xanthanes comme agents stabilisants d'émulsion huile-dans-eau comestibles, plus particulièrement des sauces piquantes de type mayonnaise par exemple. Ces émulsions se différencient des compositions nutritionnelles de l'invention par leur composition (absence d'acides aminés par exemple), par leur finalité et par le fait qu'elles ne sont pas sujettes à stérilisation.

Un objet de la présente invention est donc une composition nutritionnelle stérilisable destinée à l'alimentation entérale se présentant sous forme d'une émulsion huile-dans-eau, telle que définie à la revendication 1.

Un autre objet de la présente invention est un procédé de préparation de ladite composition nutritionnelle.

La composition ainsi préparée peut alors être stérilisée puis conditionnée de manière aseptique; la composition peut aussi être tout d'abord conditionnée puis stérilisée.

Il est aussi possible de stériliser de manière séparée l'émulsion et la solution colloïdale avant leur mélange, puis de les mélanger et de les conditionner de manière aseptique.

Les caractéristiques et avantages de la présente invention ressortiront de la description qui va suivre, dans laquelle les parties et les pourcentages sont donnés en poids.

Pour mettre en oeuvre le procédé selon l'invention, on prépare une émulsion huile-dans-eau, constituée par un mélange d'une phase aqueuse et d'une phase lipidique.

La phase lipidique peut être constituée d'huiles comestibles d'origine végétale telles que les huiles de palme, d'olive et de tournesol, ou d'origine animale telles que l'huile de beurre ou d'un mélange de ces différentes huiles, les huiles riches en acides gras polyinsaturés étant préférées.

On peut ajouter à la phase lipidique des vitamines liposolubles, telles que les vitamines A,D,E et K, ainsi que des lécithines provenant, par exemple, de l'oeuf ou du soja.

La phase aqueuse est principalement constituée d'eau, de préférence distillée ou déminéralisée, à laquelle on peut ajouter des glucides.

On a constaté que, pour maintenir correcte la stabilité de la composition nutritionnelle finale, il était préférable que les osmolalités de l'émulsion huile-dans-eau et de la solution colloïdale soient très proches, de manière à obtenir une composition finale dont l'osmolalité est, de préférence, comprise entre 250 et 1000 mOsm/kg.

Par osmolalité, on entend, dans la présente description, le nombre de moles osmotiquement actives par kilogramme de produit pris comme référence, dans le cas présent par kilogramme d'eau (unité Osm/kg d'eau).

La quantité totale de glucides souhaitée dans la composition nutritionnelle finale est donc répartie entre la phase aqueuse de l'émulsion et la solution colloïdale, de manière à maintenir proches les osmolalités de ces deux solutions, et donc renforcer la stabilité de la composition finale.

On choisit les glucides dans un groupe constitué par les mono ou polysaccharides tels que le saccharose, le maltose, le fructose ou le glucose, les maltodextrines dont l'équivalent dextrose (DE) est compris entre 10 et 50, de préférence proche de 45, et les polyalcools tels que le glycérol, le sorbitol ou le xylitol.

Afin de préparer l'émulsion huile-dans-eau tout en améliorant sa stabilité physico-chimique, on peut chauffer, de manière séparée, les phases aqueuse et lipidique jusqu'à une température de 40-55 °C, tout en maintenant une agitation constante, le chauffage étant de préférence effectué sous gaz inerte, par exemple sous azote, de manière à éviter les contaminations éventuelles ou l'oxydation des matières grasses.

On peut alors mélanger les deux phases, par exemple par addition de 10 à 45 parties de phase lipidique à 100 parties de phase aqueuse, tout en maintenant l'agitation constante et la température à une valeur de 40-55 °C.

Afin d'améliorer la stabilité de l'émulsion obtenue, on peut réduire la taille des gouttelettes de la phase lipidique, par exemple par homogénéisation.

Pour ce faire, on peut pré-homogénéiser l'émulsion sous une pression de 15-25 bar, afin d'obtenir une taille moyenne des gouttes de l'ordre de 1,5-2,5 $\mu$m, puis homogénéiser l'émulsion, par exemple dans un homogénéisateur à haute pression de l'ordre de 200-800 bar.

Cette dernière étape peut être répétée plusieurs fois, afin d'obtenir une émulsion dont la taille moyenne des gouttelettes de la phase lipidique est de l'ordre de 0,15-0,35 $\mu$m.

De préférence, l'homogénéisation est effectuée à 40 -55 °C. L'émulsion homogénéisée est ensuite maintenue sous agitation constante et sous gaz inerte tel que l'azote.

Apres homogénéisation, l'émulsion est laissée à refroidir jusqu'à température ambiante, puis neutralisée, par exemple par ajout d'hydroxyde de sodium, afin d'obtenir un pH de 7-7,4.

Parallèlement à la préparation de l'émulsion, on peut préparer une solution colloïdale comprenant une source d'acides aminés et une source de glucides.

Dans une forme particulière d'exécution, on prépare une première solution aqueuse comprenant la source d'acides aminés, une deuxième solution aqueuse comprenant la source de glucides et éventuellement une troisième solution aqueuse comprenant des vitamines et des sels minéraux puis on mélange ces différentes solutions.

La solution colloïdale comprend donc une source d'acides aminés, de préférence en solution dans de l'eau distillée ou déminéralisée.

La source d'acides aminés peut être constituée d'acides aminés, de peptides, de protéines dénaturées ou hydrolisées ou d'un mélange de ces différents composés.

La solution colloïdale comprend aussi une source de glucides. Ces glucides peuvent être des mono- ou polysaccharides tels que le saccharose, le maltose, le fructose ou le glucose, des maltodextrines dont l'équivalent dextrose est compris entre 10 et 50, de préférence proche de 45, des polyalcools ou un mélange de ces différents composés.

La quantité de glucides ajoutée dans la solution colloïdale est liée à la quantité de glucides présents dans l'émulsion, ainsi que mentionné précédemment.

Il est ensuite possible d'ajouter des sels minéraux, des vitamines et/ou des oligo-éléments, de préférence en solution aqueuse, à la solution colloïdale. Il est aussi possible d'ajouter des arômes à la solution colloïdale.

La présente composition nutritionnelle est particulièrement remarquable par le fait qu'elle comprend par partie au moins $0,6.10^{-3}$ partie de kappa-carraghénane et au moins $0,1.10^{-3}$ partie de gomme xanthane.

Le carraghénane est un polysaccharide constitué d'un mélange complexe de polymères, dont le kappa-carraghénane de formule:

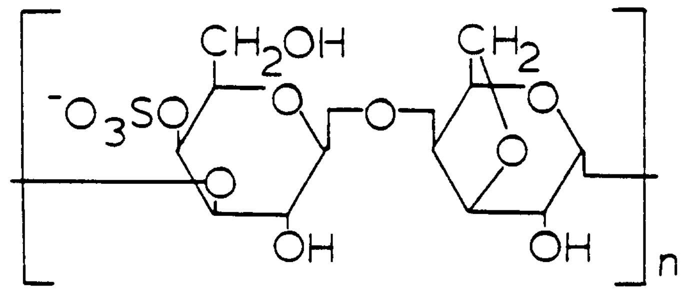

La gomme xanthane est un polysaccharide constitué d'une chaîne cellulosique à laquelle sont liés des oligosaccharides et a pour formule:

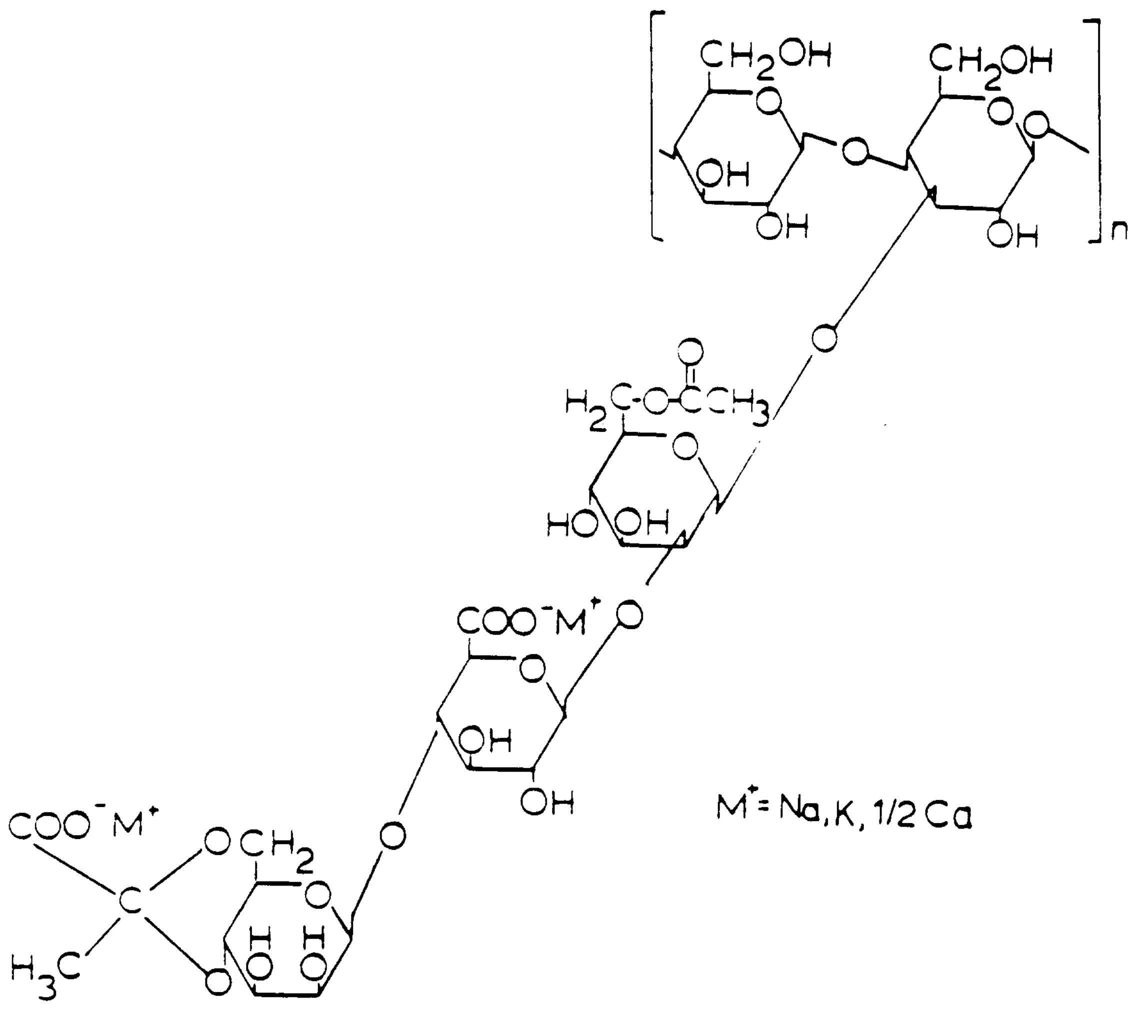

On a en effet constaté que la présence de ces constituants dans une composition se présentant sous la forme d'une émulsion huile-dans-eau permet d'assurer une bonne stabilité physique à la composition, même lorsqu'elle contient des quantités importantes d'acides aminés par exemple et/ou lorsqu'elle est ultérieurement stérilisée. Dans le cas présent, l'ajout de ces constituants à la solution colloïdale ou au mélange de l'émulsion et de la solution colloïdale permet d'améliorer la stabilité physique de la composition finale en lui conférant un comportement non-newtonien et rhéofluidifiant.

Il est possible que ces constituants particuliers améliorent la stabilité stérique de la composition, en emprisonnant les gouttelettes de lipides dans un réseau composé de chaînes peptidiques et de molécules des polysaccharides kappa-carraghénane et gomme xanthane. On ajoute donc du kappa-carraghénane et de la gomme xanthane, de manière à obtenir une composition finale comprenant par partie au moins $0,6.10^{-3}$ partie de kappa-carraghénane et au moins $0,1.10^{-3}$ partie de gomme xanthane.

De préférence, afin de conférer à la composition finale une viscosité adéquate, on n'ajoute pas plus de $1.10^{-3}$ partie de chaque constituant, par partie de composition finale.
De préférence on ajoute ces constituants en solution aqueuse, ensemble ou l'un après l'autre. On peut par exemple les ajouter à la solution colloïdale avant de la mélanger à l'émulsion; on peut également les ajouter au mélange constitué par l'émulsion et la solution colloïdale.

Ledit mélange comprend, de préférence 10 à 45% d'émulsion huile-dans-eau de pH 7-7,4 et 55-90% de solution colloïdale de pH 6,7-8,7.
On obtient ainsi une composition de pH physiologiquement acceptable et présentant une viscosité correcte qui permet l'écoulement et l'administration de ladite composition, de préférence une viscosité de l'ordre de 30-50 mPa.s (vitesse de cisaillement 50 $s^{-1}$).
Dans une forme particulière de préparation de composition stérilisée, on peut tout d'abord mélanger l'émulsion et la solution colloïdale contenant le kappa-carraghénane et la gomme xanthane, par exemple en les chauffant à 40-50 °C, sous gaz inerte, puis en maintenant une agitation constante pendant environ 30 minutes pour homogénéiser la composition formée.
On peut alors stériliser la composition, par exemple par traitement UHT, puis la conditionner, de manière aseptique, dans des boîtes, des briques ou des flacons. On peut aussi tout d'abord conditionner la composition dans des boîtes, des briques ou des flacons et les stériliser ensuite de manière appropriée.
Dans une autre forme particulière de préparation de la présente composition, on peut tout d'abord stériliser, de manière séparée, l'émulsion et la solution colloïdale, par exemple par traitement UHT, puis les mélanger aseptiquement et enfin les conditionner soit aseptiquement, soit semi-aseptiquement ce qui nécessite une stérilisation ultérieure des boîtes, briques ou flacons.
On obtient ainsi une composition nutritionnelle se présentant sous forme d'une émulsion qui peut conserver sa stabilité pendant une longue période.

De préférence, la composition nutritionnelle se présente sous forme d'une émulsion huile-dans-eau qui comprend pour une partie d'une source d'acides aminés, 0,5-7,5 parties d'une source de glucides, 0,4-1,2 parties d'une source de lipides, qui présente un taux de matière sèche de 12-38%, et qui comprend en outre par partie au moins $0,6.10^{-3}$ partie de kappa-carraghénane et au moins $0,1.10^{-3}$ partie de gomme xanthane.

La présente invention est illustrée plus en détails dans les exemples ci-après, dans lesquels les mesures sont effectuées de la manière suivante:

1) Mesure de viscosité

Effectuée à l'aide d'un rhéomètre VOR Bohlin, dans les conditions suivantes:

| | |
|---|---|
| . système de mesure | cylindre C.14 |
| . barre de torsion | 0,24 g.cm et 19,8 g.cm |
| . temps d'attente initial | 60 s |
| . temps de mesure | 20 s à déformation constante |
| . temps d'intégration | 10 s |
| . vitesse de cisaillement | 50 $s^{-1}$ ou 150 $s^{-1}$ |

2) Mesure d'osmolalité

Effectuée à l'aide d'un osmomètre Roebling, par mesure de l'abaissement cryoscopique de la solution, en prenant l'eau pure comme référence.

3) Mesure de la teneur en matière grasse

On détermine la teneur en matière grasse de la composition par mesure de la quantité de lipides, effectuée de la manière suivante:

- on prélève une quantité précise, comprise entre 10 à 20 g, de liquide à analyser,
- on dilue ce liquide avec 20 ml d'une solution aqueuse de NaCl à 10%,
- on ajuste le pH à 3 par ajout d'une solution aqueuse de HCl 0,1 N,
- on extrait quatre fois les lipides avec 50 ml d'une solution contenant 3 parties de n-hexane et 2 parties d'isopropanol,

- on lave la phase organique obtenue avec 30 ml de NaCl 10%, et la phase aqueuse obtenue avec 30 ml de n-hexane,
- les phases organiques sont réunies et séchées sur du sulfate de sodium anhydre, puis filtrées.

Les solvants organiques sont alors évaporés sous pression réduite et l'on obtient par pesée la quantité de lipides.
La teneur T en matière grasse, exprimée en %, est obtenue en faisant le rapport entre cette quantité de lipides pesée et la quantité de liquide initialement prélevé.

4) Détermination de la stabilité physique

Afin de caractériser quantitativement la stabilité physique des compositions nutritionnelles préparées, on en détermine l'indice de stabilité (IS), de la façon suivante:

- on prélève un premier échantillon de la composition préparée et l'on en détermine la teneur en matière grasse T 1;
- on prélève un second échantillon que l'on soumet à une centrifugation, à 25 °C, à 1000 tours/min. pendant 15 minutes;
- il se produit généralement une séparation en deux phases plus ou moins distinctes; on détermine la teneur en matière grasse T 2 de la partie inférieure de l'échantillon centrifugé;
- on obtient l'indice de stabilité IS (en%) en faisant le rapport

$$\frac{T\ 2 \times 100}{T\ 1}$$

Exemple 1

On prépare une émulsion huile-dans-eau en mélangeant une phase aqueuse contenant 20 g de saccharose dissous dans 110 ml d'eau déminéralisée et une phase lipidique contenant 6,5 g d'huile de maïs, 9,5 g de matière grasse lactique, 16 g de triglycérides à chaîne moyenne et 2,0 g de lécithine de soja.
Les deux phases sont préalablement chauffées à 45-50 °C et leur mélange est effectué sous azote et en maintenant une agitation constante.

L'émulsion ainsi formée est pré-homogénéisée dans un appareil adéquat, sous une pression de 20 bar et à une température de 50 °C pendant environ 30 minutes, de manière à obtenir des gouttelettes de phase lipidique présentant une taille moyenne de l'ordre de 1,5-2,5 µm.

L'émulsion préhomogénéisée est ensuite passée dans un homogénéisateur à deux étages, dont la pression totale est d'environ 500 bar, à 50 °C.

On peut recommencer cette étape plusieurs fois jusqu'à l'obtention de gouttelettes de phase lipidique présentant une taille moyenne de l'ordre de 0,2-0,3 µm.

L'émulsion est alors neutralisée jusqu'à pH 7,4 par ajout d'une solution aqueuse d'hydroxyde de sodium 0,5 N.

Parallèlement, on prépare une première solution contenant 47 g de maltodextrine de DE 11, du kappa-carraghénane et de la gomme xanthane en tant qu'agents stabilisateurs, dans les proportions indiquées ci-après et 270 ml d'eau déminéralisée.

On prépare également une deuxième solution contenant 30 g de lactalbumine dénaturée et 470 ml d'eau déminéralisée; il est possible de chauffer cette solution à 50 -80 °C pendant quelques minutes afin de faciliter la dissolution des protéines.

On prépare enfin une troisième solution contenant environ 4,0 g d'un mélange équilibré de sels minéraux comprenant des ions calcium, magnésium, sodium et potassium, dans 25 ml d'eau déminéralisée.
On mélange les trois solutions ainsi préparées, sous azote et l'on maintient l'agitation à une vitesse constante, tout en chauffant légèrement la solution colloïdale formée à une température de 45 °C environ, pendant 30 minutes, de manière à permettre aux agents stabilisateurs de remplir complètement leur fonction.

On ajoute alors l'émulsion d'osmolalité égale à 605 mOsm/kg, préalablement chauffée à 45 °C, à la solution colloïdale d'osmolalité égale à 243 mOsm/kg et de pH 6,7, sous azote, en maintenant la température à 45 °C, et l'agitation à 750 tours par minute pendant 30 minutes.

On obtient ainsi une composition nutritionnelle présentant une teneur en matière sèche de 13-14%, qui est ensuite stérilisée par traitement UHT, à 140-145 ° C pendant 3-5 secondes, puis est conditionnée de manière aseptique, dans des flacons.

Selon les quantités de kappa-carraghénane et de gomme xanthane présentes dans la composition finale, on obtient les mesures de viscosité, d'osmolalité et de pH suivantes, effectuées à 20 ° C, sur la composition stérilisée, 15 heures après sa préparation:

| | | | | | |
|---|---|---|---|---|---|
| kappa-carraghénane (g) | 0,25 | 0,50 | 0,60 | 0,75 | 1,0 |
| gomme xanthane (g) | 0,75 | 0,50 | 0,40 | 0,25 | 1,0 |
| viscosité à 50 $s^{-1}$ (mPa.s) | 32 | 15 | 55 | 55 | 116 |
| viscosité à 150 $s^{-1}$ (mPa.s) | 18 | 9,5 | 33 | 28 | 55 |
| pH | 6,1 | 6,1 | 6,6 | 6,1 | 6,3 |
| osmolalité (mOsm/kg) | 320 | 312 | 293 | 301 | 304 |

Les compositions nutritionnelles ainsi préparées restent stables et ne présentent pas de traces de séparation de phase après 15 h de stockage à une température ambiante (22-25 ° C).

Pour comparaison, on prépare de façon similaire une composition nutritionnelle contenant:

- composition A: 1,0 g de gomme xanthane
  0 g de kappa-carraghénane
- composition B: 0 g de gomme xanthane
  1,0 g de kappa-carraghénane
- composition C: 1,0 g de gomme xanthane
  1,0 g d'un mélange de iota, lambda, mu et nu-carraghénane

On observe, après préparation des compositions, et avant toute stérilisation, une séparation des phases aqueuse et lipidique, pour les trois compositions A, B et C susmentionnées.

Afin que la composition nutritionnelle reste stable, même après stérilisation, pendant au moins 15 heures, il est donc nécessaire qu'elle contienne au moins une gomme xanthane et un kappa-carraghénane.

Afin de quantifier la stabilité physique des compositions préparées, on détermine leur indice de stabilité IS.

On obtient les résultats suivants:

| | | | | | |
|---|---|---|---|---|---|
| kappa-carraghénane (g) | 0,25 | 0,50 | 0,60 | 0,75 | 1,0 |
| gomme xanthane (g) | 0,75 | 0,50 | 0,40 | 0,25 | 1,0 |
| IS (%) | 10 | 13 | 7 | 96 | 100 |

De plus, on observe après stérilisation une séparation des phases aqueuse et lipidique, pour les trois premières compositions, alors que les deux dernières restent stables.

La stabilité physique de ces deux dernières compositions est confirmée lors de leur stockage à 22-25 ° C pendant 27 semaines, pendant lequel on n'observe pas de trace de séparation de phases.

Exemple 2

On prépare de façon similaire à l'exemple 1, deux compositions comprenant 0,75 g de kappa-carraghénane et 0,25 g de gomme xanthane.

Lors de la préparation de la première composition (A), on ajoute le kappa-carraghénane et la gomme xanthane en solution aqueuse à la solution colloïdale, puis l'on mélange ladite solution colloïdale et l'émulsion.

Lors de la préparation de la seconde composition (B), on ajoute le kappa-carraghénane et la gomme xanthane en solution aqueuse à l'émulsion, puis l'on mélange ladite émulsion et la solution colloïdale.

On obtient les résultats suivants:

| | composition | |
|---|---|---|
| | A | B |
| viscosité à 50 $s^{-1}$ (mPa.s) | 55 | 48 |
| viscosité à 150 $s^{-1}$ (mPa.s) | 28 | 29 |
| pH | 6,1 | 6,6 |
| osmolalité (mOsm/kg) | 301 | 303 |
| indice de stabilité (%) | 96 | 5 |

On observe une séparation irréversible des phases aqueuse et lipidique pour la composition B alors que la composition A reste stable pendant au moins 15 heures, même après stérilisation.

Exemple 3

On prépare une émulsion en mélangeant, sous azote, a 45 °C une phase lipidique contenant 15 g de triglycérides à chaîne moyenne, 5,0 g d'huile de colza, 1,0 g de lécithine de soja et environ 0,03 g de vitamines liposolubles A,D,E et K, et 75 ml d'eau déminéralisée contenant 20 g de saccharose.

L'émulsion est homogénéisée de la même manière qu'à l'exemple 1, puis neutralisée à pH 7,4. On stérilise alors l'émulsion par traitement UHT à 140 -145 °C pendant 3-5 secondes.

Parallèlement, on prépare une solution colloïdale en mélangeant une première solution à 45 °C contenant 42 g d'acides aminés essentiels, principalement la leucine, l'isoleucine, la valine, la lysine et l'arginine, 1,0 g de vitamines parmi lesquelles les vitamines C et PP, et 300 ml d'eau déminéralisée et une seconde solution à 45 °C contenant 275 g de maltodextrine de DE 50, du kappa-carraghénane et de la gomme xanthane dans les quantités indiquées ci-après, 15 g de sels minéraux contenant Ca, Mg, K, Na et P sous forme ionique, et 250 ml d'eau déminéralisée.

La solution colloïdale ainsi formée est brassée, pendant 30 minutes, à vitesse constante, à 45 °C puis stérilisée par traitement UHT à 140-145 °C pendant 3-5 secondes.

On ajoute alors de manière aseptique la solution colloïdale stérilisée de pH 8,6 et d'osmolalité égale à 1500 mOsm/kg à l'émulsion stérilisée d'osmolalité égale à 605 mOsm/kg, sous azote en maintenant la température à 45 °C et l'agitation à 750 tours par minute pendant 30 minutes.

La composition nutritionnelle peut alors être conditionnée en flacons de manière aseptique.

On obtient une composition nutritionnelle stable et stérilisée qui présente un taux de matière sèche de 37-38%,
et les caractéristiques suivantes, mesurées à 20 ° C, sur la composition stérilisée, après 15 h de stockage à température ambiante:

| | | | | |
|---|---|---|---|---|
| kappa-carraghénane (g) | 0,25 | 0,50 | 0,75 | 1,0 |
| gomme xanthane (g) | 0,75 | 0,50 | 0,25 | 1,0 |
| viscosité à 50 $s^{-1}$ (mPa.s) | 40 | 47 | 58 | 144 |
| viscosité à 150 $s^{-1}$ (mPa.s) | 25 | 30 | 35 | 76 |
| pH | 8,8 | 8,7 | 8,8 | 8,8 |
| osmolalité (mOsm/kg) | 960 | 960 | 960 | 960 |

On remarque, dans ce cas, que l'osmolalité de la composition finale est relativement élevée, de l'ordre de 960 mOsm/kg, ce qui est dû au fait que ladite composition contient un grand nombre de particules osmotiquement actives.

La détermination de l'indice de stabilité des compositions ainsi préparées donne les résultats suivants:

| | | | | |
|---|---|---|---|---|
| kappa-carraghénane (g) | 0,25 | 0,50 | 0,75 | 1,0 |
| gomme xanthane (g) | 0,75 | 0,50 | 0,25 | 1,0 |
| IS (%) | 45 | 24 | 16 | 88 |

Exemple 4

On prépare une émulsion en mélangeant une phase aqueuse comprenant 35 g de maltodextrine de DE 11 et 250 ml d'eau déminéralisée, et une phase lipidique comprenant 15 g d'un mélange d'huile de palme, de coco et de carthame, et 1,0 g de lécithine de soja.
L'émulsion est homogénéisée de manière semblable à celle de l'exemple 1, puis neutralisée à pH 7,4.
On stérilise alors l'émulsion par traitement UHT à 140 -145 °C pendant 3-5 secondes.
Parallèlement on prépare la solution colloïdale en mélangeant 60 g de perméat de lactalbumine hydrolysée, 2,0 g de sels minéraux, du kappa-carraghénane et de la gomme xanthane comme indiqué ci-après et 640 ml d'eau déminéralisée.
La solution colloïdale est brassée à 45 °C pendant 30 minutes puis stérilisée par traitement UHT à 140-145 °C pendant 3-5 secondes.
On ajoute, de manière aseptique, l'émulsion d'osmolalité égale à 357 mOsm/kg et la solution colloïdale de pH 6,9 et d'osmolalité égale à 294 mOsm/kg, en maintenant la température à 45 °C et l'agitation à 750 tours par minute pendant 30 minutes.

La composition nutritionnelle peut être conditionnée de manière semi-aseptique puis les flacons peuvent être post-stérilisés à 120-122 °C pendant 1 minute environ.

On obtient une composition nutritionnelle stable et stérilisée qui présente un taux de matière sèche de 12-13% et les caractéristiques suivantes, mesurées à 20 °C, sur la composition stérilisée, après 15 h de stockage à température ambiante:

| kappa-caraghénane (g)<br>gomme xanthane (g) | 0,25<br>0,75 | 0,50<br>0,50 | 0,75<br>0,25 | 1,0<br>1,0 |
|---|---|---|---|---|
| viscosité à 50 $s^{-1}$ (mPa.s) | 20 | 35 | 58 | 120 |
| viscosité à 150 $s^{-1}$ (mPa.s) | 12 | 19 | 30 | 56 |
| pH | 6,2 | 6,1 | 6,0 | 6,3 |
| osmolalité (mOsm/kg) | 329 | 358 | 360 | 334 |
| IS (%) | 5 | 5 | 45 | 97 |

Les deux premières compositions présentent après stérilisation une séparation des phases aqueuse et lipidique, alors que les deux dernières compositions restent stables après stérilisation pendant au moins 27 semaines de stockage à 22-25 °C.

Exemple 5

On prépare de façon similaire à l'exemple 4 cinq compositions, dans lesquelles le kappa-carraghénane et la gomme xanthane sont ajoutés de la manière suivante:

- composition A: 0,75 g de kappa-carraghénane et
  0,25 g de gomme xanthane en solution aqueuse sont ajoutés à la solution colloïdale
- composition B: 0,75 g de kappa-carraghénane et
  0,25 g de gomme xanthane en solution aqueuse sont ajoutés au mélange constitué par la solution colloïdale et l'émulsion.
- composition C: 1,0 g de kappa-carraghénane et
  1,0 g de gomme xanthane en solution aqueuse sont ajoutés à la solution colloïdale
- composition D: 1,0 g de kappa-carraghénane et
  1,0 g de gomme xanthane en solution aqueuse sont ajoutés au mélange constitué par la solution colloïdale et l'émulsion.
- composition E: 1,0 g de kappa-carraghénane et
  1,0 g de gomme xanthane sous forme solide sont ajoutés au mélange constitué par la solution colloïdale et l'émulsion.

Les compositions sont stérilisées à 121 °C pendant 30 minutes. Pour certaines, on observe une légère séparation des phases aqueuse et lipidique, séparation qui disparaît si l'on agite légèrement la composition après sa préparation.
On mesure les caractéristiques suivantes, à 20 °C, après 15h de stockage à température ambiante:

| | composition | | | | |
|---|---|---|---|---|---|
| | A | B | C | D | E |
| viscosité à 50 $s^{-1}$ (mPa.s) | 58 | 50 | 120 | 101 | -- |
| viscosité à 150 $s^{-1}$ (mPa.s) | 30 | 30 | 56 | 54 | -- |
| pH | 6,0 | 6,7 | 6,3 | 6,7 | 6,6 |
| osmolalité (mOsm/kg) | 360 | 330 | 334 | 333 | 340 |
| IS (%) | 45 | 94 | 97 | 100 | 17 |

On observe donc que l'on peut indifféremment ajouter le kappa-carraghénane et la gomme xanthane à la solution colloïdale ou au mélange de ladite solution et de l'émulsion.
De plus, le kappa-carraghénane et la gomme xanthane sont de préférence, ajoutés sous forme de solution aqueuse.

Les exemples précédents montrent donc qu'il est possible d'obtenir une composition nutritionnelle stérilisée et stable présentant une viscosité, un pH et une osmolalité acceptables en ajoutant à ladite composition, par partie, au moins $0{,}6.10^{-3}$ partie de kappa-carraghénane et au moins $0{,}1.10^{-3}$ de gomme xanthane, et ce pour différentes sortes de compositions.

## Revendications

1. Composition nutritionnelle stérilisable destinée à l'alimentation entérale se présentant sous forme d'une émulsion huile-dans-eau, caractérisée en ce qu'elle comprend pour une partie d'une source d'acides aminés, 0,5-7,5 parties de glucides, 0,4-1,2 parties d'une source de lipides et présente un taux de matière sèche de 12 à 38%, et en ce qu'elle comprend en outre, par partie, $0{,}6\text{-}10^{-3}$ - $1.10^{-3}$ partie de kappa-carraghénane et au moins $0{,}1\text{-}10^{-3}$ - $1.10^{-3}$ partie de gomme xanthane.

2. Composition nutritionnelle selon la revendication 1, caractérisée par le fait qu'elle est stérilisée.

3. Procédé de préparation d'une composition nutritionnelle selon la revendication 1, caractérisée en ce qu'on prépare une émulsion huile-dans-eau incorporant la source de lipides, on prépare séparément une solution colloïdale incorporant la source d'acides aminés et les glucides, on ajoute à la dite solution colloïdale un kappa-carraghénane et une gomme xanthane de manière à obtenir une composition finale comprenant kappa-carraghénane et gomme xanthane dans les proportions définies à la revendication 1, puis on mélange l'émulsion et la solution colloïdale.

4. Procédé de préparation d'une composition nutritionnelle selon la revendication 1, caractérisée en ce qu'on prépare une émulsion huile-dans-eau incorporant la source de lipides, on prépare séparément une solution colloïdale incorporant la source d'acides aminés et les glucides, on mélange l'émulsion et la solution colloïdale, puis on ajoute audit mélange un kappa-carraghénane et une gomme xanthane de manière à obtenir une composition finale comprenant kappa-carraghénane et gomme xanthane dans les proportions définies à la revendication 1.

5. Procédé selon la revendication 3 ou 4, caractérisé en ce qu'on stérilise séparément l'émulsion et la solution colloïdale contenant kappa-carraghénane et gomme xanthane, puis on les mélange et les conditionne aseptiquement.

6. Procédé selon les revendications 3 ou 4, caractérisé en ce qu'on stérilise la composition finale obtenue par mélange de l'émulsion et de la solution colloïdale contenant kappa-carraghénane et gomme xanthane, puis on la conditionne de manière aseptique.

## Claims

1. A sterilizable nutritional composition in the form of an oil-in-water emulsion to be taken enterally, characterized in that it contains 0.5 to 7.5 parts of glucides and 0.4 to 1.2 parts of a lipid source to 1 part of an amino acid source and has a dry matter content of 12 to 38% and in that it additionally contains per part $0.6 \bullet 10^{-3}$ to $1.10^{-3}$ part of kappa-carrageenan and at least $0.1 \bullet 10^{-3}$ to $1.10^{-3}$ part of xanthan gum.

2. A nutritional composition as claimed in claim 1, characterized in that it is sterilized.

3. A process for the production of the nutritional composition claimed in claim 1, characterized in that an oil-in-water emulsion incorporating the lipid source is prepared, a colloidal solution incorporating the amino acid source and the glucides is separately prepared, a kappa-carrageenan and a xanthan gum are added to the colloidal solution to obtain a final nutritional composition containing kappa-carrageenan and xanthan gum in the proportions defined in claim 1, after which the emulsion and the colloidal solution are mixed.

4. A process for the production of the nutritional composition claimed in claim 1, characterized in that an oil-in-water emulsion incorporating the lipid source is prepared, a colloidal solution incorporating the amino acid source and the glucides is separately prepared, the emulsion and the colloidal solution are mixed and a kappa-carrageenan and a xanthan gum are added to the resulting mixture to obtain a final nutritional composition containing kappa-carrageenan and xanthan gum in the proportions defined in claim 1.

5. A process as claimed in claim 3 or 4, characterized in that the emulsion and the colloidal solution containing kappa-carrageenan and xanthan gum are separately sterilized and are then mixed and packed under aseptic conditions.

6. A process as claimed in claim 3 or 4, characterized in that the final composition obtained by mixing the emulsion and the colloidal solution containing kappa-carrageenan and xanthan gum is sterilized and then packed under aseptic conditions.

## Patentansprüche

1. Sterilisierbare Nährmittelzusammensetzung, die für die enterale Ernährung bestimmt ist und in Form einer Öl-in-Wasser Emulsion vorliegt, gekennzeichnet dadurch, daß sie auf einen Teil einer Aminosäurequelle 0,5-7,5 Teile Kohlenhydrate, 0,4-1,2 Teile einer Lipidquelle enthält und einen Gehalt an Trockenmasse von 12 bis 38% aufweist, und daß sie außerdem pro Teil 0,6 x $10^{-3}$ - 1 x $10^{-3}$ Teile kappa-Carrageenan und wenigstens 0,1 x $10^{-3}$ - 1 x $10^{-3}$ Teile Xanthangummi enthält.

2. Nährmittelzusammensetzung nach Anspruch 1, dadurch gekennzeichnet, daß sie sterilisiert ist.

3. Verfahren zur Herstellung einer Nährmittelzusammensetzung nach Anspruch 1, dadurch gekennzeichnet, daß man eine Öl-in-Wasser Emulsion herstellt, die die Lipidquelle enthält, getrennt davon eine kolloidale Lösung herstellt, die die Aminosäurequelle und die Kohlenhydrate enthält, daß man dieser kolloidalen Lösung ein kappa-Carrageenan und einen Xanthangummi auf eine solche Weise zugibt, daß man eine Endzusammensetzung erhält, die kappa-Carrageenan und Xanthangummi in den im Anspruch 1 angegebenen Anteilen enthält, wonach man die Emulsion und die kolloidale Lösung vermischt.

4. Verfahren zur Herstellung einer Nährmittelzusammensetzung nach Anspruch 1, dadurch gekennzeichnet, daß man eine Öl-in-Wasser Emulsion herstellt, die die Lipidquelle enthält, getrennt davon eine kolloidale Lösung herstellt, die die Aminosäurequelle und die Kohlenhydrate enthält, daß man die Emulsion und die kolloidale Lösung vermischt, dann dieser Mischung ein kappa-Carrageenan und einen Xanthangummi auf eine solche Weise zugibt, daß man eine Endzusammensetzung erhält, die kappa-Carrageenan und Xanthangummi in den im Anspruch 1 angegebenen Anteilen enthält.

5. Verfahren nach Anspruch 3 oder 4, dadurch gekennzeichnet, daß man die Emulsion und die kolloidale Lösung, die kappa-Carrageenan und Xanthangummi enthält, getrennt sterilisiert, wonach man sie vermischt und aseptisch abpackt.

6. Verfahren nach Anspruch 3 oder 4, dadurch gekennzeichnet, daß man die Endzusammensetzung sterilisiert, die durch Vermischen der Emulsion und der kolloidalen Lösung, die kappa-Carrageenan und Xanthangummi enthält, erhalten wurde, und diese auf aseptische Weise abpackt.